(19)

**Europäisches Patentamt**
**European Patent Office**
**Office européen des brevets**

(11) **EP 4 382 536 A2**

(12) **EUROPEAN PATENT APPLICATION**

(43) Date of publication:
**12.06.2024 Bulletin 2024/24**

(21) Application number: **23214883.3**

(22) Date of filing: **07.12.2023**

(51) International Patent Classification (IPC):
**C07K 16/18** (2006.01)  **G01N 33/574** (2006.01)

(52) Cooperative Patent Classification (CPC):
**C07K 16/18; G01N 33/57423;** C07K 2317/21;
C07K 2317/92

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.12.2022 US 202263386320 P**

(71) Applicants:
• **Vitae Biomedical., Co., Ltd.**
**Tapei City 115 (TW)**

• **Lee, I-Shu**
**Taichung City 402 (TW)**

(72) Inventor: **LEE, I-SHU**
**402 Taichung City (TW)**

(74) Representative: **Becker, Erin**
**Df-mp Dörries Frank-Molnia & Pohlman
Patentanwälte Rechtsanwälte PartG mbB
Theatinerstraße 16
80333 München (DE)**

Remarks:
The complete document including Reference
Table(s) and the Sequence Listing(s) can be
downloaded from the EPO website

(54) **ANTI-GM2AP ANTIBODY AND APPLICATIONS THEREOF**

(57)    The present disclosure disclosed herein a recombinant antibody or the antigen-binding fragment thereof which specifically binds GM2-activator protein (GM2AP). The recombinant antibody or the antigen-binding fragment thereof comprises a light chain variable region (LCVR) comprising three light chain complementary determining regions (LCDR1-3) amino acid sequences and a heavy chain variable region (HCVR) comprising three heavy chain complementary determining regions (HCDR1-3) set forth in the sequences disclosed in the embodiments of the present application. Polynucleotides encoding the same, vectors, host cells, kits and methods for assessing the risk of a subject suffering from lung cancer are also provided.

FIG. 2

EP 4 382 536 A2

**Description**

**REFERENCE TO SEQUENCE LISTING SUBMITTED ELECTRONICALLY**

This application contains a sequence listing, which is submitted electronically via EFS-Web as an ST.26 XML formatted sequence listing with a file name "111262-USI.xml", creation date of 2022-08-31 and having a size of 29.6 KB. The sequence listing submitted via EFS-Web is part of the specification and is herein incorporated by reference in its entirety.

**FIELD OF INVENTION**

This present disclosure relates to a novel recombinant antibody or any antigen-binding fragments thereof. Specifically, the present disclosure relates to a novel recombinant antibody or any antigen-binding fragments thereof against GM2-activator protein (GM2AP) and their applications, especially the application for assessing the risk of a subject suffering from lung cancer.

**BACKGROUND OF THE INVENTION**

Lung cancer is the leading cause of cancer deaths worldwide. Lung cancers typically start in parts of the lung and the cells lining the bronchi such as the alveoli or bronchioles. Lung cancer can be caused by smoking, breathing secondhand smoke, being exposed to substances such as asbestos or radon at home or work, having chest radiation therapy, and having a family history of lung cancer. There are two main types of lung cancer such as non-small cell lung cancer (NSCLC) and small cell lung cancer (SCLC), about 85%, and 15% of lung cancers, respectively. The main subtypes of NSCLC are squamous cell carcinoma, adenocarcinoma, and large cell carcinoma. SCLC sometimes is called oat cell cancer. The 5-year survival rate of lung cancer is only 15% due to lack of effective early detection tools and ineffective treatments for the advanced stages.

Most lung cancer patients are diagnosed in stages III and IV, and they cannot completely eradicate the tumor by surgery. Although the targeted drugs, chemotherapy, and radiotherapy have a certain therapeutic value, the long-term survival is difficult to achieve. Therefore, an effective screening test in the early-stage lung cancer is a key point to increase patient survival rate. Some of the important screening tests of lung cancer include imaging test, biomarkers, and biopsy. To reduce the death risk of lung cancer, the computed tomography (CT/LDCT) is a common screening test, but it has high false positive rate and need to be supplemented by other invasive investigations to confirm. Therefore, development of lung cancer biomarkers detection in combination with CT/LDCT screening could be a potentially useful method to increase screening accuracy and diagnose as lung cancer.

Previous studies showed that GM2-activator protein (GM2AP) expression in urine or serum from lung cancer patients is higher than healthy controls, therefore, the GM2AP could be a potential diagnostic biomarker of lung cancer. GM2AP is a small monomeric protein containing a single site for Asn linked glycosylation. It is first synthesized as a precursor which is then glycosylated, modified and cleaved at $^{32}$Ser to be in the mature form. Mature GM2AP is a glycoprotein with molecular mass of 17.6 kDa in its deglycosylated form. Acting as a cofactor, GM2AP contains at least three functional features including a hydrophobic pocket called the β-cup structure, an oligosaccharide binding site, and an area that interacts with Hex A. The area that interacts with Hex A contributes to the degradation ofGM2 ganglioside to GM3 by lysosomal β-hexminidase A (Hex A). However, only one-third of the synthesized of GM2AP is secreted. Cells can recapture the GM2AP via a carbohydrate-independent mechanism by various cells such as epidermal keratinocytes and fibroblast cells. A lack of the functional GM2AP is a cause of the abnormal accumulation ofGM2 ganglioside in tissues of patients with the AB variant of GM2 gangliosidosis disease (a severe lysosomal storage disorder). The inherited deficiency of GM2AP was also related to the changing level of ganglioside and tumor associated gangliosides involving in cancer progression. Tumor-associated gangliosides are a result of initial oncogenic transformation and play a role in the induction of invasion and metastasis. Tumor cells synthesized and shed gangliosides into their microenvironments, and this leads to elevated levels of tumor-associated gangliosides in the serum. Moreover, gangliosides are known to exhibit regulatory roles in cell growth, adhesion, cell-cell interactions and signal transduction.

**SUMMARY OF THE INVENTION**

In view of the urgent need of the art, provided herein are embodiments illustrated hereinafter.

In one embodiment, the present application provides a recombinant antibody or the antigen-binding fragment thereof which specifically binds GM2-activator protein (GM2AP), wherein the recombinant antibody or the antigen-binding fragment thereof comprises (a) a light chain variable region (LCVR) comprising three light chain complementary determining regions (LCDR1-3) amino acid sequences which are independently selected from the group consisting of SEQ ID NOs: 16-18, 22-24 and 28-30; and (b) a heavy chain variable region (HCVR) comprising three heavy chain complementary determining regions (HCDR1-3) amino acid sequences which are independently selected from the group consisting of SEQ ID NOs: 19-21, 25-27 and 31-33.

Preferably, the LCVR comprises an amino acid sequence which is selected from the group consisting of SEQ ID NOs: 4, 6 and 8, and/or the HCVR comprises an amino acid sequence which is selected from the group consisting of SEQ ID NOs: 5, 7 and 9.

More preferably, the LCVR/HCVR pair comprises the amino acid sequence pairs of SEQ ID NOs: 4/5, 6/7, 8/7, 6/9 or 8/9, respectively.

More preferably, the LCDR1/LCDR2/LCDR3/HCDR1/HCDR2/HCDR3 have amino acid sequences of SEQ ID NOs: 16/17/18/19/20/21, 22/23/24/25/26/27, 28/29/30/25/26/27, 22/23/24/31/32/33, or 28/29/30/31/32/33, respectively.

In any one of the foregoing embodiments, the recombinant antibody or the antigen-binding fragment thereof is selected from the group consisting of a diabody, a Fab, a Fab', a F(ab')$_2$, an Fv fragment, a disulfide stabilized Fv fragment (dsFv), a (dsFv)$_2$, a bispecific dsFv (dsfv-dsfv'), a disulfide stabilized diabody (ds diabody), a single-chain antibody molecule (scfv), an scfv dimer (bivalent diabody) and any combination thereof.

In any one of the foregoing embodiments, the recombinant antibody or the antigen-binding fragment thereof is selected from the group consisting of a monoclonal antibody, multispecific antibody, mouse antibody, human antibody, humanized antibody, chimeric antibody and any combination thereof.

Preferably, the recombinant antibody or the antigen-binding fragment thereof is a human antibody or a mouse antibody. Preferably, the recombinant antibody or the antigen-binding fragment thereof is a human antibody.

In one embedment, the present application provides a polynucleotide encoding any one of the recombinant antibodies or the antigen-binding fragments thereof mentioned in the present disclosure. The polynucleotide comprises a nucleic acid sequence which is selected from the group consisting of SEQ ID NOs: 10-15.

Preferably, the nucleic acid sequence encoding the LCVR is selected from the group consisting of SEQ ID NOs: 10, 12 and 14, and/or the nucleic acid sequence encoding the HCVR is selected from the group consisting of SEQ ID NOs: 11, 13 and 15.

In any one of the foregoing embodiments, the LCVR/HCVR is encoded by the nucleic acid sequence pair of SEQ ID NOs: 10/11, 12/13, 14/13, 12/15, or 14/15, respectively.

In one embedment, the present application provides a vector comprising any one of the polynucleotides mentioned in the present disclosure.

In one embedment, the present application provides a host cell comprising the vector mentioned in the present disclosure.

Preferably, the host cell is selected from the group consisting of Chinese Hamster Ovary (CHO) cell, NSO cell, BHK cell, SP2/0 cell, HEK 293 cell, HEK 293 EBNA cell, PER.C6® cell, COS cell, 239F cell, SF9 cell, SF21 cell and a combination thereof.

In one embedment, the present application provides a kit for detecting a GM2-activator protein (GM2AP) as a biomarker of lung cancer in a biological sample, wherein the kit comprises the recombinant antibody or the antigen-binding fragment thereof mentioned in the present disclosure.

Preferably, the kit further comprises a substrate, and a second antibody which conjugates with a signal generating unit and binds to a Fc domain of the recombinant antibody or the antigen-binding fragment thereof. The substrate is configured to react with the signal generating unit to generate a signal.

In any one of the foregoing embodiments, the biological sample comprises whole blood, serum, plasma, urine, or a combination thereof. Preferably, the biological sample is urine.

In any one of the foregoing embodiments, the signal generating unit comprises a radioactive marker, a fluorescent marker, a phosphorescent marker, a chemiluminescent marker or a labeling enzyme. Preferably, the labeling enzyme may comprise a horse radish peroxidase or an alkaline phosphatase.

In any one of the foregoing embodiments, the substrate is tetramethylbenzidine (TMB) when the signal generating unit is a horse radish peroxidase.

In any one of the foregoing embodiments, the kit further comprises a blocking solution. Preferably, the blocking solution comprises skim milk, bovine serum albumin, or casein.

In any one of the foregoing embodiments, the kit further comprises a wash solution. Preferably, the wash solution comprises PBS, TBS, PBS including a detergent, or TBS including a detergent.

In one embedment, the present application provides a method for assessing the risk of a subject suffering from a lung cancer, comprising the steps of: (a) determining a GM2AP content in a biological sample through the recombinant antibody or the antigen-binding fragment thereof or the kit mentioned in the present disclosure; (b) determining a total protein or creatinine content in said biological samples; (c) calculating a G/T ratio through the GM2AP content divided by the total protein or creatinine content; and (d) determining the risk of the subject suffering from a lung cancer based on the G/T ratio.

In any one of the foregoing embodiments, the biological sample comprises whole blood, serum, plasma, urine, or a combination thereof. Preferably, the biological sample is urine.

In any one of the foregoing embodiments, the step (a) further comprises the steps of: (a-1) applying the recombinant antibody or the antigen-binding fragment thereof to the biological sample; (a-2) incubating said biological sample with a secondary antibody conjugated with a detectable label; (a-3) detecting said detectable label; and (a-4) determining the GM2AP content in the biological sample based on a result of step (a-3).

In any one of the foregoing embodiments, the secondary antibody binds to a Fc domain of the recombinant antibody or the antigen-binding fragment thereof.

In any one of the foregoing embodiments, the detectable label comprises an alkaline phosphatase or a horse radish peroxidase.

In any one of the foregoing embodiments, the step (a-3) is conducted by incubating said detectable label with a tetramethylbenzidine (TMB) solution and determining an absorbance at $OD_{450}$.

In any one of the foregoing embodiments, the step (b) is conducted by detecting total protein in said biological samples and determining an absorbance at $OD_{562}$, wherein the total protein is detected by bicinchoninic acid (BCA) protein assay.

In any one of the foregoing embodiments, the step (b) is conducted by detecting total protein in said biological samples and determining an absorbance at $OD_{595}$, wherein the total protein is detected by Bradford protein assay.

In any one of the foregoing embodiments, when the G/T ratio is above 200.00 ng/mg, the subject is diagnosed to have lung cancer.

In any one of the foregoing embodiments, when the G/T ratio is between 20.00 to 200.00 ng/mg, the subject is determined to be a high risk population of lung cancer.

Preferably, when the G/T ratio is between 30.00 to 200.00 ng/mg, the subject is determined to be a high risk population of lung cancer.

More preferably, when the G/T ratio is between 30.587 to 200.00 ng/mg, the subject is determined to be a high risk population of lung cancer.

## BRIEF DESCRIPTION OF THE DRAWINGS

FIG. 1 illustrates the result of the native PAGE and SDS PAGE analysis staining with five anti-GM2AP antibody clones GBF1, GEC1, GEC2, GEC3, and GEC4.

FIG. 2 illustrates the procedures of the method for assessing the risk of a subject suffering from lung cancer.

FIG. 3 shows the standard curve of GM2AP detected by GBF1 as a capture antibody with GEC3 as the HRP conjugated detection antibody by sandwich ELISA.

FIG. 4 shows the standard curve of BSA detected by BCA protein assay with ELISA reader.

FIG. 5 show the ROC curve analysis of G/T ratio in urine samples of healthy donors and lung cancer patients. The AUC for G/T ratio in urine was 0.95 (A) at a cutoff point of 30.587 ng/mg, with a sensitivity of 100.0%, and a specificity of 85.0% (B). A line graph (C) and a dot histogram plot (D) demonstrated the distribution of G/T ratio in healthy donors and lung cancer patients in urine samples.

## DETAILED DESCRIPTION

The foregoing and other aspects of the present disclosure will now be described in more detail with respect to other embodiments described herein. It should be appreciated that the invention can be embodied in different forms and should not be construed as being limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention. As used in the description of the invention and the appended claims, the singular forms "a", "an" and "the" are intended to include the plural forms as well, unless the context clearly indicates otherwise.

As used herein, the terms "comprises," "comprising," "includes," "including," "has", "having", "contains", "containing", "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process, or method.

The transitional phrase "consisting of' excludes any elements, steps, or ingredients not specified. If in the claim, such would close the claim to the inclusion of materials other than those recited except for impurities ordinarily associated therewith. When the phrase "consisting of' appears in a clause of the body of a claim, rather than immediately following the preamble, it limits only the element set forth in that clause; other elements are not excluded from the claim as a whole.

Where applicants have defined an invention or a portion thereof with an open-ended term such as "comprising" it should be readily understood that (unless otherwise stated) the description should be interpreted to also describe such an invention using the term "consisting of".

As used herein, the term "about" is used to indicate that a value includes for example, the inherent variation of error for a measuring device, the method being employed to determine the value, or the variation that exists among the study subjects. Typically the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

The term "subject" as used herein refers to human or animals. Exemplary subject may be humans, apes, dogs, pigs, cattle, cats, horses, goats, sheep, rodents and other mammalians.

The term "antibody" as referred to herein includes intact antibodies and any antigen- binding fragment (i.e., "antigen-binding portion") or single chains thereof. An "antibody" refers to a glycoprotein comprising at least two heavy (H) chains and two light (L) chains inter-connected by disulfide bonds, or an antigen-binding portion thereof. Each heavy chain is comprised of a heavy chain variable region (abbreviated herein as VH) and a heavy chain constant region (abbreviated herein as CH). The heavy chain constant region is comprised of three domains, CH1, CH2 and CH3. Each light chain is comprised of a light chain variable region (abbreviated herein as $V_L$) and a light chain constant region. The light chain constant region is comprised of one domain, $C_L$. The $V_H$ and $V_L$ regions can be further subdivided into regions of hypervariability, termed complementarity determining regions (CDR), interspersed with regions that are more conserved, termed framework regions (FR). Each of $V_H$ and $V_L$ is composed of three CDRs and four FRs, arranged from amino-terminus to carboxy-terminus in the following order: FRI, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The "epitope" or "antigenic determinant" is a portion of an antigen molecule that is responsible for specific interactions with the antigen-binding domain of an antibody. An antigen-binding domain may be provided by one or more antibody variable domains. An antigen-binding domain can comprise at least one antibody light chain variable region (VL) and at least one antibody heavy chain variable region (VH). An antigen-binding domain can also comprise only VH or only VL regions. Specifically, in the present disclosure, the epitope could be a specific domain or a combination of domains of human GM2AP.

The term "complementarity determining regions" ("CDR") are defined as parts of the variable chains in antibodies, where these molecules bind to their specific antigen. In this disclosure, the CDR regions in the heavy chain are typically referred to as CDR-H1, CDR-H2 and CDR-H3 and in the light chain as CDR-L1, CDR-L2 and CDR-L3. They are numbered sequentially in the direction from the amino terminus to the carboxy terminus.

The extent of the framework regions and CDRs has been defined according to Kabat et al. (see, Kabat et al., Sequences of Proteins of Immunological Interest, U.S. Department of Health and Human Services, 1991) and the ImMunoGeneTics database (IMGT) (see, Lefranc, Nucleic Acids Res 29:207-9, 2001; and online at imgt.cines.fr/IMGT_vquest/vquest?livret=O&Option=humanlg).

The "Kabat system" means in the context of the present disclosure the standard for numbering the residues in a consistent manner according to Kabat (1991; Sequences of Proteins of Immunological Interest, 5th edit., NIH publication No. 91-3242U.S. Department of Health and Human Services) and Chothia (1987; J. Mol. Biol. 196, 901-917). This numbering system is widely used by the skilled artisans and is based on sequence variability and three dimensional loops of the variable domain region which are important in antigen-binding activity. All the residues of the light chains or heavy chains have distinct positions in the Kabat system; i.e., the Kabat system applies to CDRs as well as to framework regions. The positions of specific residues of any antibody may be numbered according to the Kabat system. The rules to identify the CDR regions of VH and VL chains according to Kabat system are shown in www.bioinf.org.uk/abs.

The IMGT unique numbering system is an alternative to the Kabat System that allows one to compare the variable domains whatever the antigen receptor, the chain type, or the species [Lefranc M.-P., Immunology Today 18, 509 (1997)/Lefranc M.-P., The Immunologist, 7, 132-136 (1999)/Lefranc, M.-P., Pommie, C., Ruiz, M., Giudicelli, V., Foulquier, E., Truong, L., Thouvenin-Contet, V. and Lefranc, Dev. Comp.Immunol., 27, 55-77 (2003)]. In the IMGT unique numbering system, the conserved amino acids always have the same position, for instance cysteine 23 (1st-CYS), tryptophan 41 (CONSERVED-TRP), hydrophobic amino acid 89, cysteine 104 (2nd-CYS), phenylalanine or tryptophan 118 (J-PHE or J-TRP). The IMGT unique numbering system provides a standardized delimitation of the framework regions (FRI- IMGT: positions 1 to 26, FR2-IMGT: 39 to 55, FR3-IMGT: 66 to 104 and FR4-IMGT: 118 to 128) and of the complementarity determining regions: CDR1-IMGT: 27 to 38, CDR2-IMGT: 56 to 65 and CDR3-IMGT: 105 to 117.

As gaps represent unoccupied positions, the CDR- IMGT lengths (shown between brackets and separated by dots, e.g. [8.8.13]) become crucial information. The IMGT unique numbering system is used in 20 graphical representations, designated as IMGT Colliers de Perles [Ruiz, M. and Lefranc, M.-P., Immunogenetics, 53, 857-883 (2002)/Kaas, Q. and Lefranc, M.-P., Current Bioinformatics, 2, 21-30 (2007)], and in 3D structures in IMGT/3Dstructure-DB [Kaas, Q., Ruiz, M. and Lefranc, M.-P., Tcell receptor and MHC structural data. Nucl. Acids. Res., 32, D208-D210 (2004)].

The term "antigen-binding fragment" as used herein refers to an antibody fragment, such as for example, a diabody, a Fab, a Fab', a F(ab')$_2$, an Fv fragment, a disulfide stabilized Fv fragment (dsFv), a (dsFv)$_2$, a bispecific dsFv (dsfv-dsfv'), a disulfide stabilized diabody (ds diabody), a single-chain antibody molecule (scfv), an scfv dimer (bivalent diabody), a multispecific antibody formed from a portion of an antibody comprising one or more CDRs, or any other antibody fragment that binds to an antigen but does not comprise a complete or intact antibody structure. An antigen-binding fragment is capable of binding to the same antigen to which the parent antibody or a parent antibody fragment (e.g., a parent scFv) binds. In certain embodiments, an antigen-binding fragment may comprise one or more CDRs from a particular human antibody grafted to a framework region from one or more different human antibodies. In certain embodiments, an antigen-binding fragment may comprise one or more CDRs from a particular mouse antibody grafted to a framework region from one or more different mouse antibodies.

Among the above antigen-binding fragments, a Fab, which is a structure having the light chain and heavy chain variable regions, the light chain constant region, the heavy chain first constant region (CH1), and bas one antigen-binding site. A Fab ' differs from the Fab in that the Fab' has a hinge region including at least one cysteine residue at the C-terminal of the heavy chain CH1 domain. A F(ab')$_2$ is produced when cysteine residues at the hinge region of Fab' are joined by a disulfide bond.

An Fv is a minimal antibody fragment, having only heavy chain variable region and light chain variable regions. A recombinant technique for producing the Fv fragment is well known in the art. "Single-chain Fv antibody" or "scFv" refers to an engineered antibody consisting of a light chain variable region and a heavy chain variable region connected to one another directly or via a peptide linker sequence. A two-chain Fv may have a structure in which heavy chain variable regions are linked to light chain variable regions by a non-covalent bond. A single-chain Fv may generally form a dimer structure as in the two-chain Fv, wherein heavy chain variable regions are covalently bow1d to light chain variable regions via a peptide linker or the heavy and light chain variable regions are directly linked to each other at the C-terminals thereof. The linker may be a peptide linker including any 1 to 100 or 2 to 50 amino acids, and proper sequences useful therefor are well known in the art.

The antigen-binding fragment may be obtained using a protease (for example, a whole antibody can be digested with papain to obtain Fab fragments, or can be digested with pepsin to obtain F(ab')$_2$ fragments), or may be prepared by a genetic recombinant technique.

The terms "monoclonal antibody" or "monoclonal antibody composition" as used herein refer to a preparation of antibody molecules of homogeneous molecular composition. A monoclonal antibody composition displays a single binding specificity and affinity for a particular epitope.

The term "mouse antibody," as used herein, is intended to include antibodies having variable regions in which both the framework and CDR regions are derived from murine germline immunoglobulin sequences. Furthermore, if the antibody contains a constant region, the constant region also is derived from murine germline immunoglobulin sequences. The mouse antibodies of the disclosure may include amino acid residues not encoded by murine germline immunoglobulin sequences (e.g., mutations introduced by random or site-specific mutagenesis in vitro or by somatic mutation in vivo). However, the term "mouse antibody", as used herein, is not intended to include antibodies in which CDR sequences derived from the germline of another mammalian species, such as a human, have been grafted onto mouse framework sequences.

The term "human antibody," as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the disclosure may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "mouse monoclonal antibody" refers to antibodies displaying a single binding specificity which have variable regions in which both the framework and CDR regions are derived from mouse germline immunoglobulin sequences.

The term "human monoclonal antibody" refers to antibodies displaying a single binding specificity which have variable regions in which both the framework and CDR regions are derived from human germline immunoglobulin sequences.

The term "recombinant" as used herein, is intended to include all molecules that are prepared, expressed, created or isolated by recombinant means, such as multispecific molecules (e.g. bispecific molecules) expressed using a recombinant expression vector transfected into a host cell, multispecific molecules (e.g., bispecific molecules) isolated from an animal (e.g., a mouse) that is transgenic for human immunoglobulin genes (see e.g., Taylor et al. (1992) Nucl. Acids Res. 20:6287-6295) or multispecific molecules prepared, expressed, created or isolated by any other means that involves splicing of human or mouse immunoglobulin and/or MHC gene sequences to other DNA sequences. Such recombinant multispecific molecules can include antigen-binding domains having variable and constant regions derived from human or murine germline immunoglobulin sequences.

The term "recombinant mouse antibody" as used herein, includes all mouse antibodies that are prepared, expressed, created or isolated by recombinant means, such as (a) antibodies isolated from a mouse or a hybridoma prepared therefrom (described further below), (b) antibodies isolated from a host cell transformed to express the mouse antibody, e.g., from a transfectoma, (c) antibodies isolated from a recombinant, combinatorial mouse antibody library, and (d) antibodies prepared, expressed, created or isolated by any other means that involve splicing of mouse immunoglobulin gene sequences to other DNA sequences. Such recombinant mouse antibodies have variable regions in which the framework and CDR regions are derived from mouse germline immunoglobulin sequences. In certain embodiments, however, such recombinant mouse antibodies can be subjected to in vitro mutagenesis and thus the amino acid sequences of the $V_H$ and $V_L$ regions of the recombinant mouse antibodies are sequences that, while derived from and related to mouse germline $V_H$ and $V_L$ sequences, may not naturally exist within the mouse antibody germline repertoire in vivo.

The term "recombinant human antibody" as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the $V_H$ and $V_L$ regions of the recombinant human antibodies are sequences that, while derived from and related to human germline $V_H$ and $V_L$ sequences, may not naturally exist within the human antibody germline repertoire in vivo.

The term "humanized antibody" is referred to as an antibody that is generated from non-human species and comprises protein sequences that have been modified to increase their similarity to antibody variants produced naturally in humans. The humanization process could be necessary to avoid undesired immunogenic effect when applying a non-human source antibody in human. In comparison, the term "chimeric antibody" as referred to herein is an antibody made by fusing the antigen binding region (i.e. $V_H$ and $V_L$) from one species with the constant domain with another.

The terms "percent (%) sequence identity" or "homology" are defined as the percentage of amino acid residues or nucleotides in a candidate sequence that are identical with the amino acid residues or nucleotides in the reference sequences after aligning the sequences and introducing gaps, if necessary, to achieve the maximum percent sequence identity, and excluding conservative nucleic acid substitutions. Optimal alignment of the sequences for comparison may be produced, besides manually, by means of local homology algorithms known in the art or by means of computer programs which use these algorithms (e.g., BLAST P).

The terms "bicinchoninic acid assay" or "BCA assay" are also known as "Smith assay". It is a biochemical assay for determining the total concentration of protein in a solution (0.5 $\mu$g/mL to 1.5 mg/mL). The total protein concentration is exhibited by a color change of the sample solution from green to purple in proportion to protein concentration, which can then be measured using colorimetric techniques at 562 nm.

The term "Bradford protein assay" refers to a biochemical assay for determining the total concentration of protein in a solution. Bradford protein assay is based on an absorbance shift of the dye Coomassie brilliant blue G-250. The total protein concentration is exhibited by a color change of the sample solution from brown to blue in proportion to protein concentration, which can then be measured using colorimetric techniques at 595 nm.

The nucleic acid sequences and the amino acid sequences mentioned in the following embodiments are summarized in Tables 1-4.

**Table 1. Amino Acid Sequences of GM2AP**

| SEQ ID NO: | Designation | Sequences |
|---|---|---|
| 1 | GM2AP | MQSLMQAPLLIALGLLLAAPAQAHLKKPSQLSSFSWDNCDEGKDPAVIRSLT LEPDPIIVPGNVTLSVMGSTSVPLSSPLKVDLVLEKEVAGLWIKIPCTDYIGSC TFEHFCDVLDMLIPTGEPCPEPLRTYGLPCHCPFKEGTYSLPKSEFVVPDLELP SWLTTGNYRIESVLSSSGKRLGCIKIAASLKGI |
| 2 | GM2AP | MQSLMQAPLLIALGLLLAAPAQAHLKKPSQLSSFSWDNCDEGKDPAVIRSLT LEPDPIIVPGNVTLSVVGSTSVPLSSPLKVDLVLEKEVAGLWIKIPCTDYIGSCT FEHFCDVLDMLIPTGEPCPEPLRTYGLPCHCPFKEGTYSLPKSEFVVPDLELPS WLTTGNYRIESVLSSSGKRLGCIKIAASLKGI |
| 3 | GM2AP | MQSLMQAPLLIALGLLLAAPAQAHLKKPSQLSSFSWDNCDEGKDPAVIRSLT LEPDPIIVPGNVTLSVMGSTSVPLSSPLKVDLVLEKEVAGLWIKIPCTDYIGSC TFEHFCDVLDMLIPTGEPCPEPLRTYGLPCHCPFKEGTYSLPKSEFVVPDLELP SWLTTGNYRIESVLSSSGKRLGCIKIAASLKGIRHHHHHHHH |

**Table 2. Amino Acid Sequences of the recombinant antibodies exemplified by the present application**

| SEQ ID NO: | Designation | Sequences (**Bold**: CDR1; *Italic:* CDR2; Boxed : CDR3) |
|---|---|---|
| 4 | 21-2B5-F8 LCVR | DIVMTQTASSVYVTPGESVSISCRST**KSLLHSNGDTY**LYWFLQRPGQSPQLLI *YRMS*NLAPGVPDRFSGGGSGTAFTLRISRVEAEDVGVYYC MQHLEYPFT FGA GTKLE |
| 5 | 21-2B5-F7 HCVR | EIQLQQSGAELVRPGTSVKISCMAS**GYSFTGYN**MNWVRQSHGRSLEWIGN*IN PYFGSP*NYNQKFKGKATLTLDRSSNTAYMQLNRVTSEDSAVYYC ASRQLGL GDTMDY WGQGTSVTVSS |
| 6 | 21-4E3-C81 LCVR | DIVMTQSHKFMSTSVGDRVTITCKAS**QDVSTA**VGWYQQKPGQSPKLLIY*WA STR*HTGVPDRFTGSRFGTDYTLTISSVQAEDLALYYC QQHYITPWTF GGGTK LE |
| 7 | 21-4E3-C81 HCVR | QVQLQQPGADFVKPGASVKLSCKAS**GYTFTSYW**INWVKQRPGQGLEWIGN*I YPGSGST*NYNEKFKNKATLTIDTSSSTAYMQLSSLTSDDSAVYYC SRSTLYSY DDGS WGQGTTLTVSS |
| 8 | 21-4E3-C82 LCVR | DIQMTQSPSSLSASLGERVSLTCRAS**QEISGD**LSWLQQKPDGTIKRLIY*AAS*TL ASGVPKRFSGSRSGSDYSLTISGLESEDFADYYC LQYVSYPFT FGSGTKLE |
| 9 | 21-4E3-C82 HCVR | QIQLQQSGPELGKPGASVKISCKAS**DYTFTDFY**ISWVKQRPGQGLEWIGW*IYP LDDHT*KYNEKFKGKATLTVDISSNTVYMQLSSLTSEDSAVYFC ARIYGYAMD Y WGQGTSVTVSS |

**Table 3. Nucleic Acids Sequences encoding the LCVR/HCVR of the recombinant antibodies exemplified by the present application.**

| SEQ ID NO: | Designation | Sequences |
|---|---|---|
| 10 | 21-2B5-F8 LCVR | gatattgtgatgacccagaccgcttcctccgtgtatgtgacccctggcgagtccgtgtccatttcctgccgctccaccaagtccctgctgcactccaacggcgacacctacctgtattggttcctgcagaggccaggccagtctccccagctgctgatctacagatgagcaatctggcaccaggagtgcctgacagattcagcggaggaggatccggaaccgcctttacactgcggatctctagagtggaggccgaggatgtgggcgtgtactattgtatgcagcacctggagtatcctttacctttggagccgggactaaactggaa |
| 11 | 21-2B5-F7 HCVR | gagatccagctgcagcagtctggagcagagctggtgcggccaggcacctctgtgaagatcagctgcatggcctccggctactctttcacaggctataacatgaattgggtgcggcagtcccacggcagatctctggagtggatcggcaacatcaatccctacttcggctcccctaactataatcagaagtttaagggcaaggccaccctgacactggacaggagctccaacaccgcctacatgcagctgaatcgcgtgacaagcgaggattccgccgtgtactattgtcaagcaggcagctgggactgggcgacaccatggattattggggccagggcacctccgtgacagtgtctagc |
| 12 | 21-4E3-C81 LCVR | gacatcgtgatgacccagtctcacaagttcatgtctacaagcgtgggcgacagggtgaccatcacatgcaaggcatcccaggacgtgagcaccgcagtgggatggtaccagcagaagccaggccagagccctaagctgctgatctattgggcatccaccaggcacacaggcgtgccagacagattcaccggctccagatttggcacagattacaccctgacaatcagctccgtgcaggccgaggatctggccctgtactattgtcagcagcactatatcaccccctggacatttggcggcggcacaaagctggag |
| 13 | 21-4E3-C81 HCVR | caggtgcagctgcagcagccaggagcagacttcgtgaagcctggagcctccgtgaagctgtcttgcaaggccagcggctacacctttacaagctattggatcaactgggtgaagcagaggccaggacagggactggagtggatcggcaatatctaccccggctccggctctaccaactataatgagaagttcaagaacaaggccaccctgacaatcgataccagctcctctacagcctacatgcagctgagctccctgacctctgacgatagcgccgtgtactattgtagcagatccacactgtactcctatgacgatggctcttggggccagggcaccacactgacagtgtctagc |
| 14 | 21-4E3-C82 LCVR | gacatccagatgacccagagcccaagctccctgtctgccagcctgggagagagggtgtccctgacatgcagggcatctcaggagatcagcggcgacctgtcctggctgcagcagaagcccgatggcaccatcaagcggctgatctacgcagcatctacactggcaagcggagtgcctaagcggttctccggctctagaagcggctccgattattccctgaccatctctggcctggagagcgaggactttgccgattactattgtctgcagtacgtgtcctatccccttcacctttggctctggcacaaagctggag |
| 15 | 21-4E3-C82 HCVR | cagatccagctgcagcagtctggaccagagctgggcaagcctggagcctctgtgaagatcagctgcaaggcctccgactacaccttcacagacttctacatcagctgggtgaagcagaggccaggacagggactggagtggatcggctggatctacccctggacgatcacaccaagtataacgagaagttcaagggcaaggccaccctgacagtggacatcagctccaataccgtgtacatgcagctgtctagcctgacaagcgaggactccgccgtgtactttgtgccagaatctacggctatgccatggattattggggccagggcacctccgtgacagtgtcctct |

**Table 4. Amino Acid Sequences of the recombinant antibodies exemplified by the present application**

| SEQ ID NO: | Designation | Sequences |
|---|---|---|
| 16 | 21-2B5-F8 LCDR1 | KSLLHSNGDTY |
| 17 | 21-2B5-F8 LCDR2 | RMS |
| 18 | 21-2B5-F8 LCDR3 | MQHLEYPFT |
| 19 | 21-2B5-F7 HCDR1 | GYSFTGYN |
| 20 | 21-2B5-F7 HCDR2 | INPYFGSP |
| 21 | 21-2B5-F7 HCDR3 | ASRQLGLGDTMDY |
| 22 | 21-4E3-C81 LCDR1 | QDVSTA |
| 23 | 21-4E3-C81 LCDR2 | WAS |
| 24 | 21-4E3-C81 LCDR3 | QQHYITPWTF |
| 25 | 21-4E3-C81 HCDR1 | GYTFTSYW |

(continued)

| SEQ ID NO: | Designation | Sequences |
|---|---|---|
| 26 | 21-4E3-C81 HCDR2 | IYPGSGST |
| 27 | 21-4E3-C81 HCDR3 | SRSTLYSYDDGS |
| 28 | 21-4E3-C82 LCDR1 | QEISGD |
| 29 | 21-4E3-C82 LCDR2 | AAS |
| 30 | 21-4E3-C82 LCDR3 | LQYVSYPFT |
| 31 | 21-4E3-C82 HCDR1 | DYTFTDFY |
| 32 | 21-4E3-C82 HCDR2 | IYPLDDHT |
| 33 | 21-4E3-C82 HCDR3 | ARIYGYAMDY |

Certain exemplary embodiments according to the present disclosure are described as below.

**Recombinant anti-GM2AP antibody or the antigen-binding fragment thereof**

According to one embodiment of the present disclosure, the present application provides a recombinant antibody which specifically binds GM2-activator protein (GM2AP) (the "anti-GM2AP antibody") or the antigen-binding fragment thereof. The GM2AP of this embodiment may comprise an amino acid sequence of SEQ ID NO: 1, SEQ ID NO: 2 or SEQ ID NO: 3. The anti-GM2AP antibody or the antigen-binding fragment thereof of the present embodiment may specifically bind to one or more amino acid residues within amino acids 57-70 of SEQ ID NO: 1, SEQ ID NO: 2 or SEQ ID NO: 3. Preferably, the anti-GM2AP antibody or the antigen-binding fragment thereof may specifically bind to GM2AP among amino acids 57-70 of SEQ ID NO: 1, SEQ ID NO:2 or SEQ ID NO: 3. In other words, said amino acids 57-70 of SEQ ID NO: 1, SEQ ID NO: 2 or SEQ ID NO: 3 form the epitope of the anti-GM2AP antibody or the antigen-binding fragment thereof of the present embodiment.

As used herein, an antibody that "specifically binds to GM2AP" is intended to refer to an antibody that binds to GM2AP with a binding value $K_D$ of less than $1x\ 10^{-8}$ M, less than $1 \times 10^{-9}$ M, less than $1 \times 10^{-10}$ M, less than $1 \times 10^{-11}$, or even less.

The anti-GM2AP antibody or the antigen-binding fragment thereof may comprise a light chain variable region (LCVR) and a heavy chain variable region (HCVR). In some embodiments, the LCVR may comprise an amino acid sequence having at least 80%, 85%, 90%, 91%, 92%, 93%, 94%, 95%, 96%, 97, 98, 99%, or 100% (or any percentage in between) identity to SEQ ID NO: 4, 6 or 8. Meanwhile, the HCVR may preferably comprise an amino acid sequence having at least 80%, 85%, 90%, 91%, 92%, 93%, 94%, 95%, 96%, 97, 98, 99%, or 100% (or any percentage in between) identity to SEQ ID NO: 5, 7 or 9. More preferably, the pair of LCVR and the HCVR may comprise the amino acid sequence pairs having at least 80%, 85%, 90%, 91%, 92%, 93%, 94%, 95%, 96%, 97, 98, 99%, or 100% (or any percentage in between) identity to SEQ ID NOs: 4/5, 6/7, 8/7, 6/9 or 8/9, respectively.

Moreover, said LCVR may comprise three light chain complementary determining regions (LCDR1, LCDR2 and LCDR3). Each of LCDR1, LCDR2 and LCDR3 may comprise an amino acid sequence having at least 80%, 85%, 90%, 91%, 92%, 93%, 94%, 95%, 96%, 97, 98, 99%, or 100% (or any percentage in between) identity to SEQ ID NO: 16, 17, 18, 22, 23, 24, 28, 29 or 30. Meanwhile, said HCVR may comprise three heavy chain complementary determining regions (HCDR1, HCDR2, and HCDR3). Each of HCDR1, HCDR2 and HCDR3 may comprise an amino acid sequence having at least 80%, 85%, 90%, 91%, 92%, 93%, 94%, 95%, 96%, 97, 98, 99%, or 100% (or any percentage in between) identity to SEQ ID NO: 19, 20, 21, 25, 26, 27, 31, 32 or 33.

More preferably, the LCDR1/LCDR2/LCDR3 may have the amino acid sequences having at least 80%, 85%, 90%, 91%, 92%, 93%, 94%, 95%, 96%, 97, 98, 99%, or 100% (or any percentage in between) identity to SEQ ID NOs: 16/17/18, 22/23/24 or 28/29/30, respectively. In addition, the HCDR1/HCDR2/HCDR3 may have the amino acid sequences having at least 80%, 85%, 90%, 91%, 92%, 93%, 94%, 95%, 96%, 97, 98, 99%, or 100% (or any percentage in between) identity to SEQ ID NOs: 19/20/21, 25/26/27 or 31/32/33, respectively. Still more preferably, the LCDR1/LCDR2/LCDR3/HCDR1/HCDR2/HCDR3 may have amino acid sequences having at least 80%, 85%, 90%, 91%, 92%, 93%, 94%, 95%, 96%, 97, 98, 99%, or 100% (or any percentage in between) identity to SEQ ID NOs: 16/17/18/19/20/21, 22/23/24/25/26/27, 28/29/30/25/26/27, 22/23/24/31/32/33 or 28/29/30/31/32/33, respectively.

The anti-GM2AP antibody or the antigen-binding fragment thereof, as used herein, can be a diabody, a Fab, a Fab', a F(ab')₂, an Fv fragment, a disulfide stabilized Fv fragment (dsFv), a (dsFv)₂, a bispecific dsFv (dsfv-dsfv'), a disulfide stabilized diabody (ds diabody), a single-chain antibody molecule (scfv), an scfv dimer (bivalent diabody) or any combination thereof. Moreover, the anti-GM2AP antibody or the antigen-binding fragment thereof can be a monoclonal antibody, multispecific antibody, mouse antibody, human antibody, humanized antibody, chimeric antibody and any combination thereof. Preferably, the recombinant antibody or the antigen-binding fragment thereof is a human antibody or a mouse antibody. Preferably, the recombinant antibody or the antigen-binding fragment thereof is a human antibody.

**Polynucleotides encoding the anti-GM2AP antibody or the antigen-binding fragment thereof, and vectors and host cells comprising the same**

According to one embodiment of this disclosure, the present application also provides a polynucleotide encoding any one of the anti-GM2AP antibodies or the antigen-binding fragments thereof mentioned in the present disclosure. The polynucleotide may comprise a nucleic acid sequence having at least 80%, 85%, 90%, 91%, 92%, 93%, 94%, 95%, 96%, 97, 98, 99%, or 100% (or any percentage in between) identity to SEQ ID NOs: 10-15. In some embodiment, the nucleic acid sequence encoding the LCVR of the recombinant antibody of the foregoing embodiment or the antigen-binding fragment thereof may have at least 80%, 85%, 90%, 91%, 92%, 93%, 94%, 95%, 96%, 97, 98, 99%, or 100% (or any percentage in between) identity to SEQ ID NOs: 10, 12 or 14. Meanwhile, the nucleic acid sequence encoding the HCVR of the recombinant antibody of the foregoing embodiment or the antigen-binding fragment thereof may have at least 80%, 85%, 90%, 91%, 92%, 93%, 94%, 95%, 96%, 97, 98, 99%, or 100% (or any percentage in between) identity to SEQ ID NOs: 11, 13 or 15.

Moreover, the LCVR/HCVR pair of the anti-GM2AP antibody of the foregoing embodiment or the antigen-binding fragment thereof may be encoded by the nucleic acid sequence pair having at least 80%, 85%, 90%, 91%, 92%, 93%, 94%, 95%, 96%, 97, 98, 99%, or 100% (or any percentage in between) identity to SEQ ID NOs: 10/11, 12/13, 14/13, 12/15 or 14/15, respectively.

In some embodiments, the polynucleotides are substantially identical to those coding for the amino acid sequences as described above. Substantially identical sequences may be polymorphic sequences, i.e., alternative sequences or alleles in a population. Substantially identical sequences may also comprise mutagenized sequences, including sequences comprising silent mutations. A mutation may comprise one or more nucleotide residue changes, a deletion of one or more nucleotide residues, or an insertion of one or more additional nucleotide residues. Substantially identical sequences may also comprise various nucleotide sequences that encode for the same amino acid at any given amino acid position in an amino acid sequence disclosed herein, due to the degeneracy of the nucleic acid code.

For the purposed of this disclosure, a vector comprising the polynucleotides as described above is also provided. Moreover, a host cell comprising the vector as described above is also provided. In some embodiment, the host cell can be a eukaryotic cell, preferably artificially selected or genetically engineered. The eukaryotic cell may comprise, but not be limited to, a Chinese Hamster Ovary (CHO) cell, NSO cell, BHK cell, SP2/0 cell, HEK 293 cell, HEK 293 EBNA cell, PER.C6® cell, COS cell, 239F cell, SF9 cell, SF21 cell and a combination thereof.

In general, the polynucleotides, vectors and host cells comprising the same can be used to generate the anti-GM2AP antibodies or the antigen-binding fragment thereof provided by the aforementioned embodiments. More detailed, the polynucleotides of this embodiment can be, for example, DNA or RNA and may or may not contain intronic sequences. Preferably, the polynucleotides can be a cDNA molecule.

The polynucleotides of this embodiment can be isolated and obtained by any method known in the art. For example, if the nucleotide sequence of an antibody is known, a polynucleotide encoding the antibody may be assembled from chemically synthesized oligonucleotides. This would involve, for example, the synthesis of overlapping oligonucleotides containing portions of the sequence encoding the antibody, annealing and ligating those oligonucleotides, and then amplifying the ligated oligonucleotides by PCR. The disclosed polynucleotides can also be generated from any other suitable source of nucleic acids, such as an antibody cDNA library, or a cDNA library isolated from any tissue or cells expressing the antibody (e.g., from hybridoma cells selected to express an antibody). The cDNAs encoding the light and heavy chains of the anti-GM2AP antibody made by a host cell (e.g., a hybridoma) can be obtained by standard PCR amplification or cDNA cloning techniques. For antibodies obtained from using phage display techniques, the polynucleotides encoding the anti-GM2AP antibodies provided by the aforementioned embodiments can be recovered from various phage clones of the library. In some embodiments, any of the disclosed polynucleotides or isolated nucleic acids may be incorporated into an expression vector. Suitable vectors for expression in various human and animal cell types are known in the art. In some embodiments, host cells are provided comprising the vectors. Suitable host cells include, e.g., a Chinese Hamster Ovary (CHO) cell, NSO cell, BHK cell, SP2/0 cell, HEK 293 cell, HEK 293 EBNA cell, PER.C6® cell, COS cell, 239F cell, SF9 cell, SF21 cell, and/or other human or nonhuman cell lines. In some embodiments, the host cells may transiently or stably express the polynucleotides or the isolated nucleic acids on the vector when cultured in culture medium, thereby providing a method for producing the antibodies or fragments disclosed herein.

Once the DNA fragments encoding $V_H$ and $V_L$ segments are obtained, these DNA fragments can be further manipulated by standard recombinant DNA techniques, for example to convert the variable region genes to full-length antibody chain genes, to Fab fragment genes or to an scFv gene. In these manipulations, a $V_L$- or $V_H$-encoding DNA fragment is operatively linked to another DNA molecule, or to a fragment encoding another protein, such as an antibody constant region or a flexible linker. The term "operatively linked", as used in this context, is intended to mean that the two DNA fragments are joined in a functional manner, for example, such that the amino acid sequences encoded by the two DNA fragments remain in-frame, or such that the protein is expressed under control of a desired promoter.

The isolated DNA encoding the $V_H$ region can be converted to a full-length heavy chain gene by operatively linking the $V_H$-encoding DNA to another DNA molecule encoding heavy chain constant regions (CH1, CH2 and CH3). The sequences of mouse and human (or other mammals') heavy chain constant region genes are known in the art (see e.g., Kabat, E. A., et al., (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region. In some embodiments, the heavy chain constant region is selected among IgG1 isotypes. For a Fab fragment heavy chain gene, the VH-encoding DNA can be operatively linked to another DNA molecule encoding only the heavy chain CH1 constant region.

The isolated DNA encoding the $V_L$ region can be converted to a full-length light chain gene (as well as to a Fab light chain gene) by operatively linking the $V_L$-encoding DNA to another DNA molecule encoding the light chain constant region, CL. The sequences of mouse and human (or other mammals') light chain constant region genes are known in the art (see e.g., Kabat, E. A., et al., (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or a lambda constant region.

To create an scFv gene, the $V_H$- and $V_L$-encoding DNA fragments are operatively linked to another fragment encoding a flexible linker, e.g., encoding the amino acid sequence (Gly4-Ser)$_3$ such that the $V_H$ and $V_L$ sequences can be expressed as a contiguous single-chain protein, with the $V_L$ and $V_H$ regions joined by the flexible linker (see e.g., Bird et al., 1988 Science 242:423-426; Huston et at., (1988) Proc. Natl. Acad. Sci. USA 85:5879-5883; McCafferty et al., (1990) Nature 348:552-554).

**Kits for detecting GM2AP as a biomarker of lung cancer**

According to one embodiment of the present disclosure, the present application also provides a kit for detecting a GM2-activator protein (GM2AP) in a biological sample. The kit may comprise the anti-GM2AP antibody or the antigen-binding fragment thereof mentioned in the present disclosure. Preferably, the kit of this embodiment may further comprise a substrate and a second antibody which conjugates with a signal generating unit and binds to a Fc domain of the anti-GM2AP antibody or the antigen-binding fragment thereof. The substrate is configured to react with the signal generating unit to generate a signal. Moreover, the kit may further comprise a blocking solution and/or a wash solution. Preferably, the blocking solution may comprise skim milk, bovine serum albumin, or casein and the wash solution may comprise PBS, TBS, PBS including a detergent, or TBS including a detergent.

In some embodiments, the biological sample may comprise whole blood, serum, plasma, urine, or a combination thereof. Preferably, the biological sample is urine. In addition, the signal generating unit may comprise a radioactive marker, a fluorescent marker, a phosphorescent marker, a chemiluminescent marker or a labeling enzyme. Preferably, the labeling enzyme may comprise a horse radish peroxidase or an alkaline phosphatase.

More preferably, the substrate may be tetramethylbenzidine (TMB) provided that the signal generating unit is a horse radish peroxidase.

In some embodiments, the kit may further detecting total protein content or creatinine content.

In any one of the foregoing embodiments, the kits for detecting GM2AP content and/or total protein content or creatinine content includes but not limited to ELISA kit, biosensor, biochip, and rapid test.

**Methods for assessing the risk of a subject suffering from lung cancer**

The present application also provides a method for assessing the risk of a subject suffering from a lung cancer by detecting a GM2-activator protein (GM2AP) in a biological sample by the foregoing anti-GM2AP antibody or the antigen-binding fragment thereof, or more specifically, by the kit of the preceding embodiment. The method of this embodiment may the following steps:

(a) determining a GM2AP content in a biological sample through the recombinant antibody or the antigen-binding fragment thereof or the kit mentioned in the present disclosure;
(b) determining a total protein or creatinine content in said biological samples;
(c) calculating a G/T ratio through the GM2AP content divided by the total protein or creatinine content; and

(d) determining the risk of the subject suffering from a lung cancer based on the G/T ratio.

In some embodiments, the step (a) further comprises the steps of:

(a-1) applying the recombinant antibody or the antigen-binding fragment thereof to the biological sample;
(a-2) incubating said biological sample with a secondary antibody conjugated with a detectable label;
(a-3) detecting said detectable label; and
(a-4) determining the GM2AP content in the biological sample based on a result of step (a-3).

For the purpose of the present disclosure, the biological sample may comprise whole blood, serum, plasma, urine, or a combination thereof, and the secondary antibody may bind to a Fc domain of said anti-GM2AP antibody or the antigen-binding fragment thereof. Preferably, the biological sample is urine. In some embodiments, the detectable label may comprise an alkaline phosphatase. Meanwhile, the step (a-3) can be conducted by incubating said detectable label with ap-nitrophenyl phosphate solution and determining an absorbance at $OD_{405}$.

Alternatively, the detectable label may comprises a horse radish peroxidase and the step (a-3) can be conducted by incubating said detectable label with a tetramethylbenzidine (TMB) solution and determining an absorbance at $OD_{450}$.

In some embodiments, the step (b) may conducted by detecting total protein in said biological samples and determining an absorbance at $OD_{562}$, wherein the total protein is detected by bicinchoninic acid (BCA) protein assay.

Alternatively, the step (b) may conducted by detecting total protein in said biological samples and determining an absorbance at $OD_{595}$, wherein the total protein is detected by Bradford protein assay.

In some embodiments, when the G/T ratio is above 200.00 ng/mg, the subject is diagnosed to have lung cancer. Alternatively, when the G/T ratio is between 20.00 to 200.00 ng/mg, the subject is determined to be a high risk population of lung cancer. Preferably, when the G/T ratio is between 30.00 to 200.00 ng/mg, the subject is determined to be a high risk population of lung cancer. More preferably, when the G/T ratio is between 30.587 to 200.00 ng/mg, the subject is determined to be a high risk population of lung cancer.

It will be apparent to those skilled in the art that various modifications and variations can be made to the disclosed embodiments. It is intended that the following experimental examples are considered as exemplary only.

### Experimental Examples

All materials used in the following experimental examples are commercially available or can be easily obtained by the person with ordinary skills in the art from related commercially available materials without undue experiments.

### Production and purification of anti-human GM2AP mouse antibody

The human *GM2AP* genes were derived from NCBI protein databank. The amino acid sequences of GM2AP are listed in Table 1. In the present application, GM2AP or a peptide fragment of the GM2AP was i.p. injected into BALB/C mice to generate monoclonal antibodies. Specifically, in an embodiment, the GM2AP (SEQ ID NO: 1 or SEQ ID NO: 2) linked with $(GlcNAc)_2Fuc(Man)_3$ was used to induce antibodies in a test subject. In another embodiment, the GM2AP tagged with poly(His), such as eight Histidines, (SEQ ID NO: 3) was used to induce antibodies in a test subject for the convenience of purification. Other tags, such as FLAG, Myc, HA, etc., may be applied as well.

To generate monoclonal antibodies specifically recognizing GM2AP, briefly, the recombinant GM2AP (SEQ ID NO: 3) was expressed in SF9 cells and/or SF21 cells. The expressed recombinant GM2AP was purified by Ni Excell column (Buffer A: 0.3M NaCl, 25 mM sodium phosphate, 20 mM Imidazole, pH8.0; Buffer B: 0.3M NaCl, 25mM sodium phosphate, 500 mM Imidazole, pH8.0; dialysis Buffer: 0.3M NaCl, 25mM sodium phosphate, pH8.0). The SF21 cell lysate was first filtered through 0.22 uM membrane. The Ni Excell column was pre-equilibrated with Buffer A followed by loading the filtered cell lysate onto column. The column was washed with Buffer A and the recombinant GM2AP was eluted from the column with 4%, 8%, 16%, 50% and 100% of buffer B. The 16% and 8% fractions were harvested and dialyzed in dialysis Buffer.

The purified GM2AP was i.p. injected into BALB/C mice to generate monoclonal mouse antibodies. Afterwards, the mice were sacrificed and the activated splenocytes were fused with melanoma cells to generate hybridomas. The hybridoma clones which showed the highest affinities to the GM2AP among all were i.p. injected into mice for ascites production to amplify the antibodies. The antibody clones which showed the highest affinity to human GM2AP among all were further selected for further investigation, for example, clone No. 21-2B5-F7, 21-2B5-F8, 21-4E3-C81, 21-4E3-C82, etc.

Further, mouse antibodies of clone No. 21-2B5-F7, 21-2B5-F8, 21-4E3-C81 and 21-4E3-C82 were subject to sequence analysis. The amino acid sequences of the LCVR/HCVR and the corresponding CDRs (LCDR1-3 and HCDR1-3) are shown in Table 2. The nucleic acids sequences encoding the LCVR/HCVR of these monoclonal antibodies are shown in Table 3.

**Production and purification of the recombinant anti-GM2AP antibody**

The nucleic acid and amino acid sequences of LCDRs, HCDRs, LCVRs and HCVRs of the recombinant anti-GM2AP antibody which specifically bind GM2AP are listed in Table 2-4.

In the present application, the recombinant anti-GM2AP antibodies were codon optimized and synthesized for expression in mammalian cells with pcDNA3.4-TOPO (Thermo Fisher Scientific) vector, which was transfected into Expi293 cells (Thermo Fisher Scientific). Other suitable host cells included but not limited to 239F cell, BHK cell, Chinese Hamster Ovary (CHO) cell, COS cell, HEK 293 cell, HEK 293 EBNA cell, PER.C6® cell, NSO cell, SP2/0 cell, SF9 cell, SF21 cell and a combination thereof. The transfected cells were grown in 1 L culture medium at 37 °C, 125 rpm, 8% $CO_2$ atmosphere. The transfected cells were harvested at 144 hours post transfection and centrifuged at 2,000×g for 1 h at 4 °C. The supernatant was then filtered through a 0.22 $\mu$m filter.

The recombinant anti-GM2AP antibody can rapidly and reliably be purified with a protein G affinity column (GE Healthcare). The filtered supernatant was loaded on the protein G column following the manufacture's instruction manual with using PBS to equilibrate the column (binding buffer) and 0.7% acetic acid (elution buffer) to elute the antibodies. The elution was monitored by measuring absorbance at 280 nm.

**Native and SDS-PAGE analysis**

The recombinant anti-GM2AP antibody (5 $\mu$g) was diluted in a loading buffer without dithiothreitol (DTT) or a sample buffer with 0.55 M $\beta$-mercaptoethanol (BME), added in each lane of 10% native polyacrylamide gel electrophoresis (PAGE) or sodium dodecyl sulfate (SDS) PAGE, and stained with 0.25% Coomassie blue at room temperature (RT) for 30 min and de-stained with $ddH_2O$ at RT for 2 hr. The recombinant anti-GM2AP antibodies included GBF1, GEC1, GEC2, GEC3, and GEC4 (listed in Table 5 below).

**Table 5. Anti-GM2AP antibodies**

| Antibodies | LCVR/HCVR |
| --- | --- |
| GBF1 | 21-2B5-F8 LC/21-2B5-F7 HC |
| GEC1 | 21-4E3-C81 LC/21-4E3-C81 HC |
| GEC2 | 21-4E3-C82 LC/21-4E3-C81 HC |
| GEC3 | 21-4E3-C81 LC/21-4E3-C82 HC |
| GEC4 | 21-4E3-C82 LC/21-4E3-C82 HC |

**Preparation of capture antibodies**

The recombinant anti-GM2AP antibodies, such as GBF1, GEC1, GEC2, GEC3, or GEC4, were diluted in a coating buffer (0.05 M carbonate-bicarbonate buffer, pH 9.6, Sigma) to prepare the final concentration of 5 $\mu$g/mL coating reagent. 100 $\mu$L of coating reagent was added in each well of 96-well plate. After sticked with a sealing film (Bersing), the 96-well plate was incubated at 4 °C for 16 to 20 hrs. After incubation, the fluid in each well was completely removed. And then each well was washed by a wash buffer for several times. Finally, the plate was sticked with a sealing film for storage at -20 °C.

**Preparation of detection antibodies**

Horseradish peroxidase (HRP) was conjugated to detection antibody with HRP conjugation Kit (Abcam). 50 $\mu$g of the purified anti-GM2AP antibody (such as GBF1, GEC1, GEC2, GEC3, or GEC4) was mixed with 5 $\mu$L of modifier reagent and 100 $\mu$g of HRP mix to produce a conjugation mixture. The conjugation reaction of the conjugation mixture was then quenched according to manufacturer's instruction. The HRP conjugated detection antibody was mixed with glycerol for storage at -20 °C. In addition, the signal generating unit may comprise a radioactive marker, a fluorescent marker, a phosphorescent marker, a chemiluminescent marker or a labeling enzyme. Preferably, the labeling enzyme may comprise a HRP or an alkaline phosphatase.

**Collection of the biological sample**

Human urine samples from 100 healthy donors and 100 lung cancer patients were obtained from Taiwan. Table 6 demonstrated the study population including healthy donors and patients with lung cancer. The urine samples of healthy group showed 58 male and 42 female with a mean age of 50 (range, 20-79 years old), and patients also showed 58 male and 42 female with a mean age of 69 (range, 38-93 years old). All urine samples were collected 10 mL in a collecting tube. Then, the urine samples were subsequently centrifuged at $1,000 \times g$ for 5 min at 4°C to remove cellular contamination and debris. The supernatants were collected in 15 mL sterilized centrifuge tubes and stored at -80 °C until further analysis. In addition, the biological sample may comprise whole blood, serum, plasma, or a combination thereof.

**Methods for detecting and calculating the G/T ratio.**

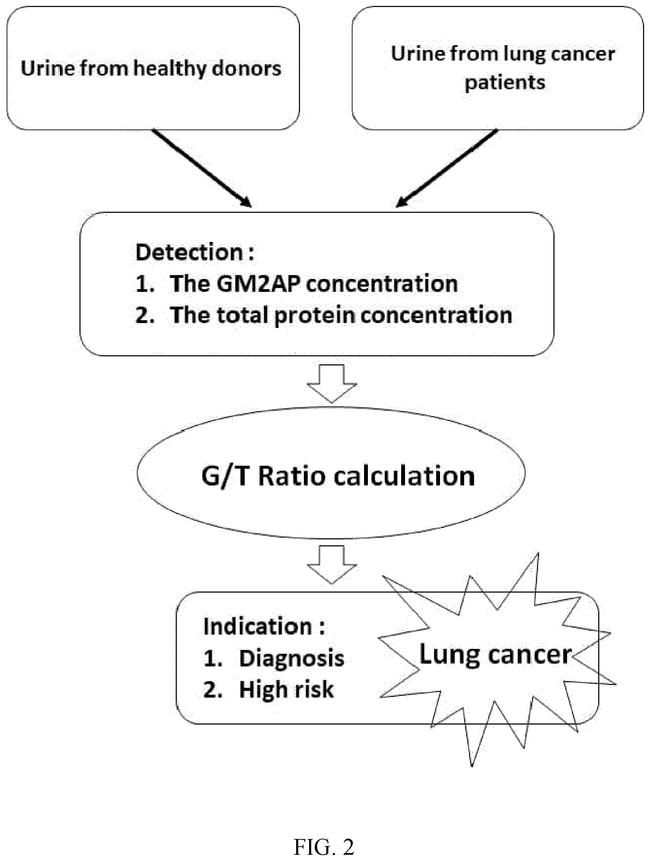

In this example, a method for detecting and calculating the G/T ratio in urine samples or other biological samples by the recombinant anti-GM2AP antibody is provided. The method comprises the following steps: (a) applying the recombinant antibodies produced above to a biological sample; (b) incubating the same biological sample with a secondary antibody conjugated with a detectable label; (c) detecting the detectable label; (d) determining the GM2AP content and the total protein or creatinine content in the same biological sample; and (e) calculating G/T ratio through the GM2AP content divided by the total protein or creatinine content. FIG. 2 demonstrates the collection of the human urine samples and the abovementioned method for detecting and calculating the G/T ratio.

**Detection GM2AP in urine with sandwich ELISA.**

Sandwich ELISA was carried out with 96-well plate, which was coated with 0.5 $\mu$g of purified capture antibody for each well at RT for 10 min. After blocking, the human urine sample was diluted by 10-fold to 200-fold dilution and added to each well coated with capture antibody at 37 °C for 1 h. After washing, 0.42 $\mu$g of HRP conjugated detection antibody (100 $\mu$L per well) was added to each well. The colour was developed by adding 100 $\mu$L tetramethylbenzidine (TMB, Thermo Fisher Scientific) to each well for 10 min before adding 100 $\mu$L of 2N sulfuric acid (sigma) per well to stop the chromogenic reaction before the absorbance at 450 nm was measured. The recombinant GM2AP was diluted by 2-fold serial dilution. Using the same capture-detection antibody pair and quantity, and the same 96-well plate to undergo sandwich ELISA to calculate the amount of GM2AP in human urine sample. In addition, the capture-detection antibody pair has 20 pairing combinations to detect the amount of GM2AP in human or mammalian biological sample.

**Bicinchoninic acid protein assay for detecting the total protein in urine.**

Bicinchoninic acid (BCA) protein assay was carried out with ELISA by 96-well plate following the Pierce BCA Protein Assay Kit (Thermo Fisher Scientific) manufacture's instruction manual. Bovine serum albumin (BSA) as a standard was diluted by 2-fold serial dilution to quantify and produce a linear response curve with the absorbance at 562 nm was measured. This response curve allowed accurate determination of total protein concentration. The human urine sample was diluted by 10-fold to 200-fold dilution by BCA protein assay and corresponded with a linear response curve of BSA to calculate the amount of total proteins in human urine sample. Another protein assay is Bradford protein assay, which also used BSA as a standard to quantify and produce a linear response curve with the absorbance at 595 nm was measured.

**Calculation of the G/T ratio**

In the same biological sample, a G/T ratio is calculated by the following equation.

$$\text{G/T ratio} = \frac{[\text{the GM2AP content in a biological sample}]}{[\text{the total protein content in the same biological sample}]}$$

The G/T ratio can be used as an indicator for identifying the high risk population of lung cancer or for the diagnosis of lung cancer. The clinical data of G/T ratio can be further processed with the statistical analysis. In addition, the G/T ratio can also be calculated by replacing the total proteins content with the creatinine content as shown in the following equation.

$$\text{G/T ratio} = \frac{[\text{the GM2AP content in a biological sample}]}{[\text{the creatinine content in the same biological sample}]}$$

**Statistical analysis**

All data were shown as mean $\pm$ standard error of the mean (SEM). Statistical analysis was performed using the easyROC. Receiver-operating characteristic (ROC) curves display the cut-off between sensitivity and specificity for G/T ratio differentiating between patients and healthy donors. The correlation between G/T ratio and lung cancer patients was evaluated by Mann-Whitney U Test. Thep values less than 0.05 were considered as statistically significant.

**Results**

*Production of the recombinant anti-GM2AP antibodies*

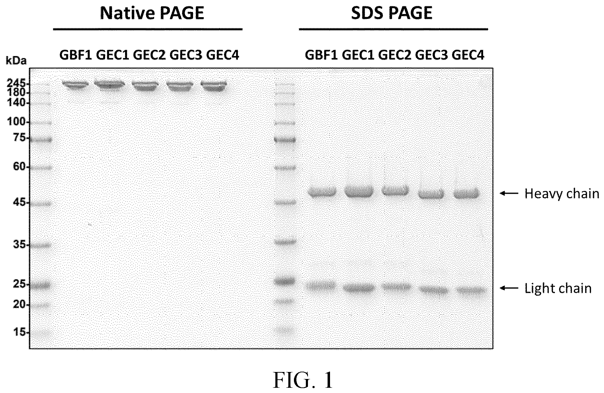

The five recombinant antibodies (GBF1, GEC1, GEC2, GEC3, and GEC4, listed in Table 5) all comprise a light chain variable region (LCVR) comprising three light chain complementary determining regions (LCDR1-3) sequences and a heavy chain variable region (HCVR) comprising three heavy chain complementary determining regions (HCDR1-3) sequences (Table 2-4). In this example, the light chain constant region of all recombinant antibodies is a mouse kappa constant region, and the heavy chain constant region of all recombinant antibodies is linkage with a mouse IgG1. To confirm the recombinant anti-GM2AP antibody production and purification, the five recombinant antibodies (GBF1, GEC1, GEC2, GEC3, and GEC4, listed in Table 5) are analysis by native PAGE and SDS-PAGE. FIG. 1 shows GBF1, GEC1, GEC2, GEC3, and GEC4 bands on native PAGE and SDS PAGE. As shown in FIG. 1, the molecular weights of the light chain and the heavy chain of GBF1 are 24.3 and 49.0 kDa, respectively; the molecular weights of the light chain of GEC1 and GEC3 both are 23.9 kDa; the molecular weights of the light chain of GEC2 and GEC4 both are 23.6 kDa; the molecular weights of the heavy chain of GEC1 and GEC2 both are 48.7 kDa; and the molecular weights of the heavy chain of GEC3 and GEC4 both are 48.8 kDa.

*Detection of GM2AP and total protein in urine samples*

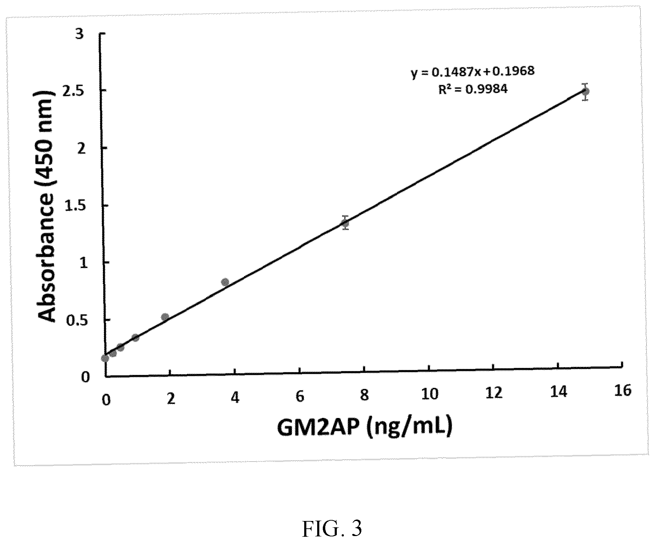

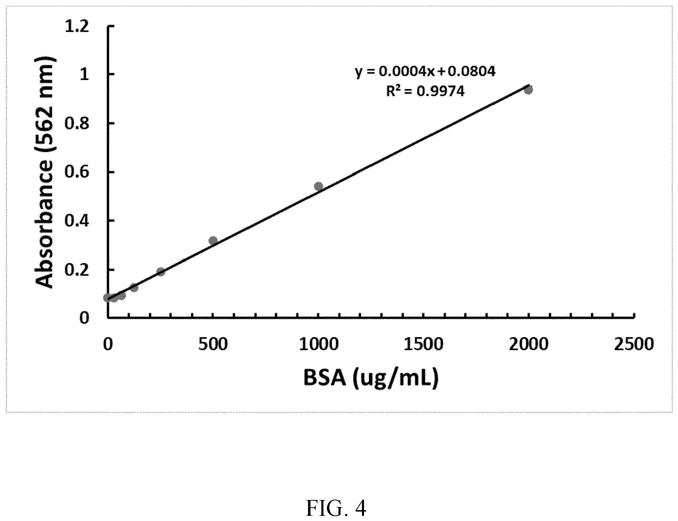

The recombinant GM2AP was diluted by 2-fold serial dilution to be detected with ELISAs assay, including but not limited to direct, indirect, sandwich, and competitive types. Preferably, the sandwich ELISA seems to have higher sensitivity and specificity than other types. In this example, 20 different capture-detection antibody pairs were used to detect the recombinant GM2AP content. FIG.3 demonstrates a standard curve of GM2AP detected by GBF1 as a capture antibody with GEC3 as a HRP conjugated detection antibody, and this standard curve can be used for calculating GM2AP concentration in urine sample from healthy donors and lung cancer patients.
In addition, BSA was diluted by 2-fold serial dilution to be detected by BCA protein assay with ELISA reader. FIG.4 demonstrates a standard curve of BSA for calculating total protein concentration in urine sample from healthy donors and lung cancer patients.

*G/T ratio in urine sample.*

The G/T ratios in urine samples obtained from 100 healthy donors and 100 lung cancer patients were determined through an ELISA assay. Table 6 demonstrates that the mean of G/T ratio in all lung cancer patients was 92.61$\pm$61.3 ng/mg and 15.52$\pm$22.2 ng/mg in all healthy donors. The results suggests that the average G/T ratio in urine from healthy male are not statistically different to those from healthy female ($p > 0.05$), but the average G/T ratio in urine from male patient are a significantly higher than those from female patient ($p < 0.05$). In addition, there was a 5.97$\pm$2.8 folds increase of G/T ratio in urine compared with those obtained from healthy donors.

Table 6. Distribution in urine levels of G/T ratio.

| | G/T ratio (ng/mg) | | |
| | Urine | | |
| **Parameters** | **n** | **Mean $\pm$ SD** | **p Value** |
| Healthy donors | | | |
| *Age* | | | |
| Median, 50 yrs (range, 20-79) | 100 | 15.52$\pm$22.2 | |

(continued)

| | G/T ratio (ng/mg) | | |
| --- | --- | --- | --- |
| | Urine | | |
| Parameters | n | Mean ± SD | *p* Value |
| *Gender* | | | |
| Male | 58 | 16.52±23.7 | 0.5969 |
| Female | 42 | 14.13±20.3 | |
| Patients with lung cancer | | | |
| *Age* | | | |
| Median, 69 yrs (range, 38-93) | 100 | 92.61±61.3 | $3.36\times10^{-115}$ |
| *Gender* | | | |
| Male | 58 | 104.80±68.3 | 0.0187 |
| Female | 42 | 75.77±45.8 | |

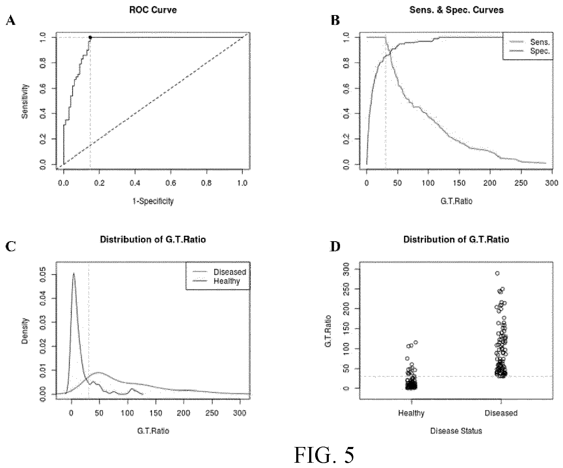

ROC curve analysis of G/T ratio in urine samples of healthy donors and lung cancer patients is shown in FIG. 5, and these results prediction are 95% correct has an AUC of 0.949 at a cutoff point of 30.587 ng/mg that provided 100.0% sensitivity and 85.0% specificity. As shown in FIG. 5D, there were 15 urine samples from healthy donors whose G/T ratio were above the cutoff point, especially one of them having a G/T ratio of 115.99 ng/mg. On the other hand, Table 6 showed that there were 25 urine samples from male patient whose G/T ratio were above the 104.80 ng/mg, and 15 urine samples from female patient whose G/T ratio were above the 75.77 ng/mg. As shown in FIG. 5D, the 9 urine samples from patients whose G/T ratio were all above the 200.00 ng/mg. These results suggested a significant increase of G/T ratio in urine of lung cancer patients ($p < 0.05$). Therefore, the G/T ratio can be used as two indicators, one is for diagnosing lung cancer when the ratio is higher than 200.00 ng/mg, and the other is for identifying the high risk population of lung cancer when the ratio is higher than a predetermined cutoff point (e.g., 30.587 ng/mg).

This new detection method can assist any medical images, including but not limited to CT, LDCT, X-ray, magnetic resonance imaging (MRI), positron emission tomography (PET), and a combination thereof, to screen and diagnose as lung cancer in clinical diagnosis of potential patients. In addition, this new method also can apply in diverse biological samples, such as whole blood, serum, plasma, or a combination thereof, and develop various screen and detection kits, but not limited to ELISA kit, biosensor, biochip, and rapid test.

Without further elaboration, it is believed that one skilled in the art can, based on the above description, utilize the present invention to its fullest extent. The following specific examples are, therefore, to be construed as merely illustrative, and not limitative of the remainder of the disclosure in any way whatsoever.

All references (e.g., publications or patents or patent applications) cited herein are incorporated herein by reference in their entirety and for all purposes to the same extent as if each individual reference (e.g., publication or patent or patent application) was specifically and individually indicated to be incorporated by reference in its entirety for all purposes. Other embodiments are within the following claims.

ENUMERATED EXAMPLES:

The following Enumerated Examples set forth below provide additional aspects of the present disclosure.

Example 1. An recombinant antibody or the antigen-binding fragment thereof which specifically binds GM2-activator protein (GM2AP), wherein the recombinant antibody or the antigen-binding fragment thereof comprises:

a light chain variable region (LCVR) comprising three light chain complementary determining regions (LCDR1-3); and a heavy chain variable region (HCVR) comprising three heavy chain complementary determining regions (HCDR1-3); wherein the LCDR1/LCDR2/LCDR3/HCDR1/HCDR2/HCDR3 comprises an amino acid sequence which is selected from the group consisting of SEQ ID NOs: 16/17/18/19/20/21, 22/23/24/25/26/27, 28/29/30/25/26/27, 22/23/24/31/32/33, and 28/29/30/31/32/33.

Example 2. The recombinant antibody or the antigen-binding fragment thereof according to Example 1, wherein the LCVR comprises an amino acid sequence which is selected from the group consisting of SEQ ID NOs: 4, 6 and 8 and the HCVR comprises an amino acid sequence which is selected from the group consisting of SEQ ID NOs: 5, 7 and 9.

Example 3. The recombinant antibody or the antigen-binding fragment thereof according to Example 1 or 2, comprising:

the LCVR comprising an amino acid sequence of SEQ ID NO: 4 and the HCVR comprising an amino acid sequence of SEQ ID NO: 5;
the LCVR comprising an amino acid sequence of SEQ ID NO: 6 and the HCVR comprising an amino acid sequence of SEQ ID NO: 7;
the LCVR comprising an amino acid sequence of SEQ ID NO: 8 and the HCVR comprising an amino acid sequence of SEQ ID NO: 7;
the LCVR comprising an amino acid sequence of SEQ ID NO: 6 and the HCVR comprising an amino acid sequence of SEQ ID NO: 9; or
the LCVR comprising an amino acid sequence of SEQ ID NO: 8 and the HCVR comprising an amino acid sequence of SEQ ID NO: 9.

Example 4. The recombinant antibody or the antigen-binding fragment thereof according to any one of Examples 1-3, wherein when the LCDR1/LCDR2/LCDR3/HCDR1/HCDR2/HCDR3 is 22/23/24/25/26/27, the LCVR comprises an amino acid sequence of SEQ ID NO: 6, when the LCDR1/LCDR2/LCDR3/HCDR1/HCDR2/HCDR3 is 28/29/30/31/32/33, the LCVR comprises an amino acid sequence of SEQ ID NO: 8.

Example 5. The recombinant antibody or the antigen-binding fragment thereof according to any one of Examples 1-4, wherein:

the recombinant antibody or the antigen-binding fragment thereof is selected from the group consisting of a diabody, a Fab, a Fab', a F(ab')$_2$, an Fv fragment, a disulfide stabilized Fv fragment (dsFv), a (dsFv)$_2$, a bispecific dsFv (dsfv-dsfv'), a disulfide stabilized diabody (ds diabody), a single-chain antibody molecule (scfv), an scfv dimer (bivalent diabody) and any combination thereof; or
the recombinant antibody or the antigen-binding fragment thereof is selected from the group consisting of a monoclonal antibody, multispecific antibody, mouse antibody, human antibody, humanized antibody, chimeric antibody and any combination thereof.

Example 6. A kit for detecting a GM2-activator protein (GM2AP) as a biomarker of lung cancer in a biological sample, comprising the recombinant antibody or the antigen-binding fragment thereof according to any one of Examples 1-5.

Example 7. The kit according to Example 6, further comprises a substrate, and a second antibody which conjugates with a signal generating unit and binds to a Fc domain of the recombinant antibody or the antigen-binding fragment thereof.

Example 8. The kit according to Example 6 or 7, wherein the biological sample comprises whole blood, serum, plasma, urine, or a combination thereof.

Example 9. The kit according to Example 7 or 8, wherein the signal generating unit comprises a radioactive marker, a fluorescent marker, a phosphorescent marker, a chemiluminescent marker or a labeling enzyme.

Example 10. The kit according to Example 9, wherein the labeling enzyme comprises a horse radish peroxidase or an alkaline phosphatase.

Example 11. The kit according to any one of Examples 7-10, wherein the substrate is tetramethylbenzidine (TMB) when the signal generating unit is a horse radish peroxidase.

Example 12. The kit according to any one of Examples 6-11, the kit further comprises a blocking solution, the blocking solution comprises skim milk, bovine serum albumin, or casein.

Example 13. The kit according to any one of Examples 6-12, the kit further comprises a wash solution, the wash solution comprises PBS, TBS, PBS including a detergent, or TBS including a detergent.

Example 14. A method for assessing the risk of a subject suffering from a lung cancer, comprising:

(a) determining a GM2AP content in a biological sample through the recombinant antibody or the antigen-binding fragment thereof according to any one of Examples 1-5 or the kit according to any one of Examples 6-13;
(b) determining a total protein or creatinine content in said biological samples;
(c) calculating a G/T ratio through the GM2AP content divided by the total protein or creatinine content; and
(d) determining the risk of the subject suffering from a lung cancer based on the G/T ratio.

Example 15. The method according to Example 14, wherein the biological sample comprises whole blood, serum, plasma, urine, or a combination thereof.

Example 16. The method according to Example 14 or 15, wherein the step (a) further comprises the steps of:

(a-1) applying the recombinant antibody or the antigen-binding fragment thereof to the biological sample;
(a-2) incubating said biological sample with a secondary antibody conjugated with a detectable label;

(a-3) detecting said detectable label; and

(a-4) determining the GM2AP content in the biological sample based on a result of step (a-3).

Example 17. The method according to Example 16, wherein the secondary antibody binds to a Fc domain of the antibody or the antigen-binding fragment thereof.

Example 18. The method according to Example 16 or 17, wherein the detectable label comprises an alkaline phosphatase or a horse radish peroxidase.

Example 19. The method according to any one of Examples 16-18, wherein the step (a-3) is conducted by incubating said detectable label with a tetramethylbenzidine (TMB) solution and determining an absorbance at $OD_{450}$.

Example 20. The method according to any one of Examples 14-19, wherein the step (b) is conducted by detecting total protein in said biological samples and determining an absorbance at $OD_{562}$, wherein the total protein is detected by bicinchoninic acid (BCA) protein assay.

Example 21. The method according to any one of Examples 14-19, wherein the step (b) is conducted by detecting total protein in said biological samples and determining an absorbance at $OD_{595}$, wherein the total protein is detected by Bradford protein assay.

Example 22. The method according to any one of Examples 14-21, wherein when the G/T ratio is above 200.00 ng/mg, the subject is diagnosed to have lung cancer.

Example 23. The method according to any one of Examples 14-21, wherein when the G/T ratio is between 20.00 to 200.00 ng/mg, the subject is determined to be a high risk population of lung cancer.

Example 24. The method according to Example 23, wherein when the G/T ratio is between 30.587 to 200.00 ng/mg, the subject is determined to be a high risk population of lung cancer.

**Claims**

1. An recombinant antibody or the antigen-binding fragment thereof which specifically binds GM2-activator protein (GM2AP), wherein the recombinant antibody or the antigen-binding fragment thereof comprises:

   a light chain variable region (LCVR) comprising three light chain complementary determining regions (LCDR1-3); and
   a heavy chain variable region (HCVR) comprising three heavy chain complementary determining regions (HCDR1-3);
   wherein the LCDR1/LCDR2/LCDR3/HCDR1/HCDR2/HCDR3 comprises an amino acid sequence which is selected from the group consisting of SEQ ID NOs: 16/17/18/19/20/21, 22/23/24/25/26/27, 28/29/30/25/26/27, 22/23/24/31/32/33, and 28/29/30/31/32/33.

2. The recombinant antibody or the antigen-binding fragment thereof according to claim 1, wherein the LCVR comprises an amino acid sequence which is selected from the group consisting of SEQ ID NOs: 4, 6 and 8 and the HCVR comprises an amino acid sequence which is selected from the group consisting of SEQ ID NOs: 5, 7 and 9.

3. The recombinant antibody or the antigen-binding fragment thereof according to claim 2, comprising:

   the LCVR comprising an amino acid sequence of SEQ ID NO: 4 and the HCVR comprising an amino acid sequence of SEQ ID NO: 5;
   the LCVR comprising an amino acid sequence of SEQ ID NO: 6 and the HCVR comprising an amino acid sequence of SEQ ID NO: 7;
   the LCVR comprising an amino acid sequence of SEQ ID NO: 8 and the HCVR comprising an amino acid sequence of SEQ ID NO: 7;
   the LCVR comprising an amino acid sequence of SEQ ID NO: 6 and the HCVR comprising an amino acid sequence of SEQ ID NO: 9; or
   the LCVR comprising an amino acid sequence of SEQ ID NO: 8 and the HCVR comprising an amino acid sequence of SEQ ID NO: 9.

4. The recombinant antibody or the antigen-binding fragment thereof according to claim 1, wherein:

   the recombinant antibody or the antigen-binding fragment thereof is selected from the group consisting of a diabody, a Fab, a Fab', a F(ab')$_2$, an Fv fragment, a disulfide stabilized Fv fragment (dsFv), a (dsFv)$_2$, a bispecific dsFv (dsfv-dsfv'), a disulfide stabilized diabody (ds diabody), a single-chain antibody molecule (scfv), an scfv dimer (bivalent diabody) and any combination thereof; or
the recombinant antibody or the antigen-binding fragment thereof is selected from the group consisting of a monoclonal antibody, multispecific antibody, mouse antibody, human antibody, humanized antibody, chimeric antibody and any combination thereof.

5. A kit for detecting a GM2-activator protein (GM2AP) as a biomarker of lung cancer in a biological sample, comprising the recombinant antibody or the antigen-binding fragment thereof according to claim 1, optionally wherein the biological sample comprises whole blood, serum, plasma, urine, or a combination thereof.

6. The kit according to claim 5, further comprises a substrate, and a second antibody which conjugates with a signal generating unit and binds to a Fc domain of the recombinant antibody or the antigen-binding fragment thereof, optionally wherein the signal generating unit comprises a radioactive marker, a fluorescent marker, a phosphorescent marker, a chemiluminescent marker or a labeling enzyme.

7. The kit according to claim 6, wherein the labeling enzyme comprises a horse radish peroxidase or an alkaline phosphatase, optionally wherein the substrate is tetramethylbenzidine (TMB) when the signal generating unit is a horse radish peroxidase.

8. The kit according to claim 5, the kit further comprises a blocking solution, the blocking solution comprises skim milk, bovine serum albumin, or casein, optionally wherein the kit further comprises a wash solution and the wash solution comprises PBS, TBS, PBS including a detergent, or TBS including a detergent.

9. A method for assessing the risk of a subject suffering from a lung cancer, comprising:

(a) determining a GM2AP content in a biological sample through the recombinant antibody or the antigen-binding fragment thereof according to claim 1 or the kit according to claim 6;
(b) determining a total protein or creatinine content in said biological samples;
(c) calculating a G/T ratio through the GM2AP content divided by the total protein or creatinine content; and
(d) determining the risk of the subject suffering from a lung cancer based on the G/T ratio.

10. The method according to claim 9, wherein the step (a) further comprises the steps of:

(a-1) applying the recombinant antibody or the antigen-binding fragment thereof to the biological sample;
(a-2) incubating said biological sample with a secondary antibody conjugated with a detectable label;
(a-3) detecting said detectable label; and
(a-4) determining the GM2AP content in the biological sample based on a result of step (a-3).

11. The method according to claim 10, wherein the secondary antibody binds to a Fc domain of the antibody or the antigen-binding fragment thereof.

12. The method according to claim 10, wherein the detectable label comprises an alkaline phosphatase or a horse radish peroxidase, optionally wherein the biological sample comprises whole blood, serum, plasma, urine, or a combination thereof.

13. The method according to claim 10, wherein the step (a-3) is conducted by incubating said detectable label with a tetramethylbenzidine (TMB) solution and determining an absorbance at $OD_{450}$.

14. The method according to claim 9, wherein the step (b) is conducted by detecting total protein in said biological samples and determining an absorbance at $OD_{562}$, wherein the total protein is detected by bicinchoninic acid (BCA) protein assay; or wherein the step (b) is conducted by detecting total protein in said biological samples and determining an absorbance at $OD_{595}$, wherein the total protein is detected by Bradford protein assay.

15. The method according to claim 9, wherein when the G/T ratio is above 200.00 ng/mg, the subject is diagnosed to have lung cancer.

FIG. 1

FIG. 2

FIG. 3

FIG. 4

**A** ROC Curve

**B** Sens. & Spec. Curves

**C** Distribution of G.T.Ratio

**D** Distribution of G.T.Ratio

FIG. 5

# EP 4 382 536 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **KABAT et al.** Sequences of Proteins of Immunological Interest. *U.S. Department of Health and Human Services,* 1991
- **LEFRANC.** *Nucleic Acids Res,* 2001, vol. 29, 207-9
- **KABAT.** Sequences of Proteins of Immunological Interest. Department of Health and Human Services, 1991
- **CHOTHIA.** *J. Mol. Biol.,* 1987, vol. 196, 901-917
- **LEFRANC M.-P.** *Immunology Today,* 1997, vol. 18, 509
- **LEFRANC M.-P.** *The Immunologist,* 1999, vol. 7, 132-136
- **LEFRANC, M.-P. ; POMMIE, C. ; RUIZ, M. ; GIUDI-CELLI, V ; FOULQUIER, E ; TRUONG, L. ; THOU-VENIN-CONTET, V.** *Lefranc, Dev. Comp.Immunol.,* 2003, vol. 27, 55-77
- **RUIZ, M ; LEFRANC, M.-P.** *Immunogenetics,* 2002, vol. 53, 857-883
- **KAAS, Q ; LEFRANC, M.-P.** *Current Bioinformatics,* 2007, vol. 2, 21-30
- **KAAS, Q. ; RUIZ, M. ; LEFRANC, M.-P.** Tcell receptor and MHC structural data. *Nucl. Acids. Res,* 2004, vol. 32, D208-D210
- **TAYLOR et al.** *Nucl. Acids Res,* 1992, vol. 20, 6287-6295
- **KABAT, E. A. et al.** Sequences of Proteins of Immunological Interest. U.S. Department of Health and Human Services, 1991
- **BIRD et al.** *Science,* 1988, vol. 242, 423-426
- **HUSTON.** *Proc. Natl. Acad. Sci. USA,* 1988, vol. 85, 5879-5883
- **MCCAFFERTY et al.** *Nature,* 1990, vol. 348, 552-554